# EUROPEAN PATENT APPLICATION

(11) **EP 3 824 816 A1**
(43) Date of publication of application: **26.05.2021**
(21) Application number: 19211195.3
(22) Date of filing: 25.11.2019
(51) Int. Cl.: A61B 7/00, A61B 7/02, A61B 7/04

(54) **SYSTEM AND METHOD FOR DETECTING LUNG ABNORMALITIES**

(71) Applicant: Koninklijke Philips N.V., 5656 AG Eindhoven (NL)
(72) Inventor: HIWALE, Sujitkumar Sureshrao, 5656 AE Eindhoven (NL); CHAKRABARTI, Biswaroop, 5656 AE Eindhoven (NL); BERA, Deep, 5656 AE Eindhoven (NL); JOHNSON, Mark Thomas, 5656 AE Eindhoven (NL)
(74) Representative: Philips Intellectual Property & Standards

(57) **Abstract**

A system is provided for detecting lung abnormalities in a subject. The system comprises a first sensor, for sensing a first acoustic signal generated by, or applied to, the subject and an arrangement of one or more second sensors for detecting a plurality of second acoustic signals from the lungs of the subject, wherein the second acoustic signals comprise attenuated versions of the first acoustic signal after passing through the lungs of the subject. The system further comprises a processor, wherein the processor is configured to determine a respective signal attenuation between the first acoustic signal and at least a subset of the plurality of second acoustic signals and process the signal attenuations thereby to detect a lung abnormality.

## Description

### FIELD OF THE INVENTION

This invention relates to the field of detecting lung abnormalities. It relates in particular to a system for determining a signal attenuation caused by a lung abnormality.

### BACKGROUND OF THE INVENTION

Pathology of the respiratory system is one of the most common reasons for visits to outpatient departments. The respiratory system consists of a respiratory tract and lungs. The lungs are connected to the upper airways (the trachea, which in turn branches into bronchi) and comprise various levels of branching bronchi and bronchioles (lobar bronchi, segmental bronchi, sub-segmental bronchi, conducting bronchiole, terminal bronchiole, respiratory bronchiole, alveolar duct and alveolar sac) and ultimately alveoli. Gas exchange takes place through the walls of the alveoli. As the larynx is situated at the beginning of trachea, any sound voiced by the patient is transmitted through the airways and may be heard over the chest and back using a stethoscope and physicians indeed take advantage of this phenomenon while auscultating for 'whispered pectoriloquy', bronchophony and egophony during auscultation. Analysis of lung (respiratory) sounds is an active area of research and has been explored by researchers for a long time. The literature covers various techniques that are currently being used to collect and analyze respiratory sounds. However, variations in intensity and quality of respiratory sounds from patient to patient make it often difficult to objectively quantify changes in the sound properties.

The change in characteristics of transmitted sounds is used to diagnose certain conditions such as presence of fluid or solid mass in lung fields. The patient is asked to pronounce some pre-chosen word (e.g. 'ninety-nine', long 'e' etc.) at a given loudness (whisper in case of whispered pectoriloquy, normal conversation loudness for bronchophony and egophony) and the physician listens for any characteristic changes in the sound. Normally it is not possible to detect the whispered sound by auscultation, but would be audible in case of an underlying lung consolidation (i.e. collection of fluid). In lung consolidation the sounds generated in the larger airways are transmitted more efficiently through the consolidated region, so they are louder in intensity and there is a better transmission of higher frequencies. Similarly, for bronchophony, consolidation may be detected by absence of expected normal decay of loudness and clarity of the sound in peripheral region of the lung or change in bilateral symmetry or contrasting loudness compared to neighboring locations. On the other hand, presence of fluid or pleural effusion/pneumothorax will have respiratory sounds with intensity lower than expected. Doctors also observe whether there is any deviation in the attenuation of higher and lower frequency components of the pronounced sounds (e.g. increased attenuation of higher frequency components may cause a pronounced long 'e' to be perceived as an 'a' sound).

Nevertheless, conventional clinical methods that utilize vocal sound transmission through airways for bedside diagnosis have a few important limitations: subjectivity in assessment; difficulty in examining two sides of chest wall simultaneously; difficulty in using these methods in ambulatory conditions; requirement of a quiet setting; inability to detect small sized lesions/pathologies; subjectivity in sound production (vocalization); and inability in using conventional techniques in unconscious or uncooperative patients

Therefore, there is a need for a system which can reliably and objectively determine lung abnormalities.

### SUMMARY OF THE INVENTION

The invention is defined by the claims.

According to examples in accordance with an aspect of the invention, there is provided a system for detecting lung abnormalities in a subject comprising:
a first sensor for sensing a first acoustic signal generated by, or applied to, the subject;
an arrangement of one or more second sensors for detecting a plurality of second acoustic signals from the lungs of the subject, wherein the second acoustic signals comprise attenuated versions of the first acoustic signal after passing through the lungs of the subject; and
a processor for detecting lung abnormalities, wherein the processor is configured to:
   determine a respective signal attenuation between the first acoustic signal and at least a subset of the plurality of second acoustic signals; and
   process the signal attenuations thereby to detect a lung abnormality.

The system uses a first sensor to sense an acoustic signal from the subject. The acoustic signal may be a speech produced by the subject, a sound produced in the body of the subject or a signal externally generated and transmitted through the body of the subject. The acoustic signal then is transmitted throughout the body of the subject. An arrangement of second sensors is placed near the lungs of the subject to detect a plurality of second acoustic signals after they have been transmitted, and attenuated, throughout the lungs. A processor then determines how much the second acoustic signals have been attenuated compared to the acoustic signal. A lung abnormality can then be detected based on how much the signal has been attenuated.

Two main implementation options are possible.

In a first option, the arrangement of second sensors is provided having just a single second sensor, or a small patch of second sensors, and the second sensor/patch is manually moved to different positions on the subject's chest to (sequentially) acquire second acoustic signals at different locations across the chest. This set of second acoustic signals corresponding to the different locations is then received by the processor which processes the signals as described above.

In a second option, the arrangement of second sensors comprises a plurality of second sensors, or patches of second sensors. The second sensors are arranged such that, when the apparatus is in place on a subject's chest, each second sensor is positioned at a different location across the chest of the subject. Each can therefore receive a second acoustic signal at a different location across the chest.

An acoustic actuator may generate the first acoustic signal, wherein the acoustic actuator is for application in the vicinity of the larynx of the subject, and wherein the first sensor is adapted to sense the first acoustic signal directly or to sense the first acoustic signal based on a drive signal provided to the acoustic actuator.

Alternatively, the first acoustic signal may comprise speech generated by the subject, wherein the first sensor is one of: a standalone microphone, a throat microphone, an accelerometer, a wave radar, a Doppler radar or an ultrasound sensor.

The processor may be configured to detect a presence or absence of speech in the first acoustic signal based on frequency and intensity analysis of the first acoustic signal, and is further configured to identify predetermined words or word sequences.

The first acoustic signal may be noisy or include external sounds which are not transmitted through the body, for example an ambulance siren. By determining the presence or absence of speech in the first acoustic signal before any further analysis, the likelihood of misleading results is reduced.

The processor may comprise a trained machine learning speech recognition network for identifying said predetermined words or word sequences.

The processor may be further configured to:
determine the placement of the one or more second sensors based on the plurality of second acoustic signals, wherein the placement comprises rib-alignment or intercostal space-alignment;
determine a subset of the one or more second sensors that are intercostal space-aligned; and
determine a subset of the plurality of second acoustic signals generated by the subset of the one or more second sensors that are intercostal space-aligned.

The ribs and intercostal spaces between the ribs cause respectively different effects on the intensity of sounds transmitted though the lungs, with the ribs causing attenuation of sounds from within the chest and augmentation of sounds originating from the surface of the chest, while the intercostal spaces cause little or no attenuation or augmentation of such sounds. As they both produce different changes in the signal, it is desirable to classify the signals by whether they are aligned with the ribs or with the intercostal spaces.

The processor may be configured to:
determine the signal attenuation, at least for said subset of the plurality of second acoustic signals, as a ratio between the intensity of the respective second acoustic signal and the intensity of the first acoustic signal, for a same word or word sequence.

As the signals from the intercostal spaces have minimal attenuation or augmentation, it may be desirable to only use the signals which have been sensed form the intercostal spaces for consistency.

The processor may be configured to:
determine a vocal resonance level, at least for said subset of the plurality of second acoustic signals, thereby to identify a type or range of possible types of lung abnormality.

It is known that different vocal resonance levels (such as normal resonant, increased vocal resonant, diminished vocal resonant and absent vocal resonant) may be used to provide an indication of a type (or possible set of types) of lung abnormality.

The processor may be configured to:
determine a frequency spectrum of the first acoustic signal and at least said subset of the plurality of second acoustic signals;
normalize the amplitudes of the frequency spectra of the first and second acoustic signals; and
perform cross correlations between components of the normalized frequency spectrum of the first acoustic signal and the corresponding components of the normalized frequency spectra of the second acoustic signals.

In this way, an attenuation function with respect to frequency may be derived. The way different frequency components are attenuated may also indicate the presence or type of lung abnormality.

The processor may be further configured to:
for a plurality of lung regions, determine clusters of the second sensors which are associated with each lung region, based on the placement of each of the second sensors, wherein a second sensor is associated with the lung regions to which it is closest; and
for a detected lung abnormality, determine the position of the lung abnormality based on the clusters of second sensors at which the lung abnormality is detected.

This enables a location of a detected abnormality to be identified.

The processor may be further configured to:
classify at least each of said second sensors corresponding to said subset of the plurality of second acoustic signals as associated with a central part of a lung region or a peripheral part of a lung region; and
compare the second acoustic signals between a sensor classified as associated with the central part of a lung region and a sensor classified as associated with the peripheral part of that lung region.

By processing central and peripheral parts of lung regions separately, the accuracy of detection may be further improved.

The processor may comprise a machine learning model trained using a database of sounds in respect of normal and abnormal pathologies, and in respect of sensor signals for central parts of the lung regions and for peripheral parts of the lung regions.

The processor may be configured to compare second acoustic signals for opposite sides of the chest at least for said subset of the plurality of second acoustic signals.

The invention also provides a method for determining a lung abnormality likelihood indicator, wherein the lung abnormality likelihood indicator indicates the likelihood of a subject having a lung abnormality, the method comprising:
receiving signals from a first sensor for sensing a first acoustic signal generated by, or applied to, the subject;
receiving signals from an arrangement of one or more second sensors for detecting a plurality of second acoustic signals from the lungs of the subject, wherein the second acoustic signals comprise attenuated versions of the first acoustic signals after passing through the lungs of the subject; and
determining the lung abnormality likelihood indicator by:
   determining respective signal attenuations between the first acoustic signal and at least a subset of the plurality of second acoustic signals; and
   processing the signal attenuations thereby to determine the lung abnormality likelihood indicator.

The invention also provides a computer program comprising code means for implementing the method for determining a lung abnormality likelihood indicator when said computer program is run on a processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a first example of a system used to detect lung abnormalities;
Figure 2 shows a second example of a system used to detect lung abnormalities which uses a standalone microphone;
Figure 3 shows a third example of a system used to detect lung abnormalities which uses an actuator;
Figure 4 shows an example of an arrangement of sensors;
Figure 5 shows a representation of human lungs;
Figure 6 shows a fourth example of a system used to detect lung abnormalities which uses a microphone array and an acoustic actuator;
Figure 7 shows a first example of how a lung abnormality may be detected;
Figure 8 shows an example of how a processor may calculate the signal attenuation;
Figure 9 shows an example of how to determine if a pulmonary segment has an abnormality; and
Figure 10 shows an example of how to categorize the attenuated acoustic signals.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for detecting lung abnormalities in a subject. The system comprises a first sensor, for sensing a first acoustic signal generated by, or applied to, the subject and an arrangement of one or more second sensors for detecting a plurality of second acoustic signals from the lungs of the subject, wherein the second acoustic signals comprise attenuated versions of the first acoustic signal after passing through the lungs of the subject. The system further comprises a processor, wherein the processor is configured to determine a respective signal attenuation between the first acoustic signal and at least a subset of the plurality of second acoustic signals and process the signal attenuations thereby to detect a lung abnormality.

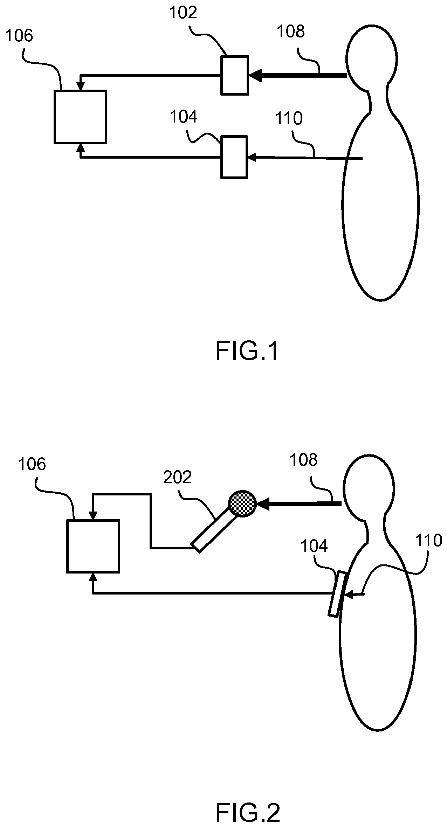

Figure 1 shows a first example of a system used to detect lung abnormalities. The system uses a first sensor 102 to sense a first acoustic signal 108 from the subject. The first acoustic signal 108 may be a speech produced by the subject, a sound produced in the body of the subject or a signal externally generated and transmitted through the body of the subject. The first acoustic signal 108 is then transmitted throughout the body of the subject. An arrangement of second sensors 104 is placed near the lungs of the subject to detect second acoustic signals after they have been transmitted, and attenuated, throughout the lungs. A processor 106 then determines how much the second acoustic signal 110 has been attenuated compared to the first acoustic signal 108.

A processor 106 is used in order to make the analysis of the first acoustic signal 108 objective and improve accuracy in the diagnosis of pulmonary conditions.

To solve the limitations of conventional methods, both the sound production (at or near the larynx) and the sound reception at multiple points on the chest are quantified using the first sensor 102 and the arrangement of sensors 104.

For example, the arrangement of second sensors 104 could be a patch, for positioning over the chest wall of the subject, comprising of one or more microphones. Furthermore the first sensor 102 could be either a standalone microphone to quantify the word spoken by the subject or an acoustic actuator (loudspeaker) to create a standardized acoustic input signal. The first sensor 102 could also be an arrangement of microphones or any combination of sensors which could detect the acoustic signal 108.

The arrangement of second sensors 104 could be used to record the sound/vibration transmitting through the chest wall. Then, either the subject is asked to say a few specific words (e.g. ninety-nine) and the subject's voice is recorded using a standalone microphone, or an acoustic actuator creates a sound close to the position of the larynx of the subject.

The arrangement of second sensors 104 could be provided having just a single second sensor, or a small patch of second sensors, and the second sensor/patch is manually moved to different positions on the subject's chest (or back) to sequentially acquire attenuated (second) acoustic signals 110 at different locations across the chest or back. This set of attenuated acoustic signals 110 corresponding to the different locations is then received by the processor which processes the signals and determines the signal attenuation.

The arrangement of second sensors 104 could also comprise a plurality of second sensors, or a plurality of patches of second sensors. The second sensors could be arranged such that, when the apparatus is in place on a subject's chest (or back), each second sensor is positioned at a different location across the chest of the subject. Each can therefore receive the second acoustic signals 110 at a different location across the chest.

In another example, the system could involve use of Doppler radar technology, wave radar or other similar technologies for noncontact measurement of either of the acoustic signal 108 or the attenuated (second) acoustic signal 110.

Figure 2 shows a second example of a system used to detect lung abnormalities which uses a standalone microphone 202. The standalone microphone 202 would pick up a first acoustic signal 108 from the subject, such as speech. An arrangement of second sensors 104 can then pick up the signals which have been transmitted through the body of the subject from the chest (or back). The transmitted signals will have been attenuated by the body. A processor 106 can then compare the signals from the standalone microphone 202, the first acoustic signal 108, and the signals from the arrangement of sensors 104, the second acoustic signal 110. The signals should be similar in shape and frequency spectrum when there is no lung abnormality. A lung abnormality can cause a change in the second acoustic signal 110 when measured from the chest (or back). The changes can be detected by the processor 106 in order to detect a lung abnormality.

In an example, an arrangement of second sensors 104 comprising a single microphone can be used to listen to sounds transmitted through subject's chest along with a standalone microphone 202 placed close to the subject's mouth. The single microphone can be then used to get measurements from the different part of the chest wall (or back) in a pre-defined sequence. These measurements are then processed and compared to create a chest (or back) surface map.

The standalone microphone 202 is a common device which can be easily operated, therefore simplifying the detection of lung abnormalities.

One issue of using a standalone microphone 202 is that it can also pick up background sounds which may limit the accuracy of the measurements. For this reason, it may be desirable to use a throat microphone or laryngophone instead of standalone microphone. A throat microphone is a type of microphone which directly captures vibrations from a throat instead of air. The throat microphone can acquire speech even in extremely noisy conditions, which can be beneficial in situations with high levels of background noise.

In an example of how a throat microphone may be used, a subject is first asked to say a few specific words. The sounds transmitting through throat are then picked by throat microphone and the arrangement of second sensors 104 is used to detect sounds over the chest wall (or back). The two sounds are then processed to detect any lung pathologies.

Vocal cord vibration can be also detected using other modalities such as an accelerometer placed on the throat to detect the vibrations, or an ultrasound device which can detect the vibrations.

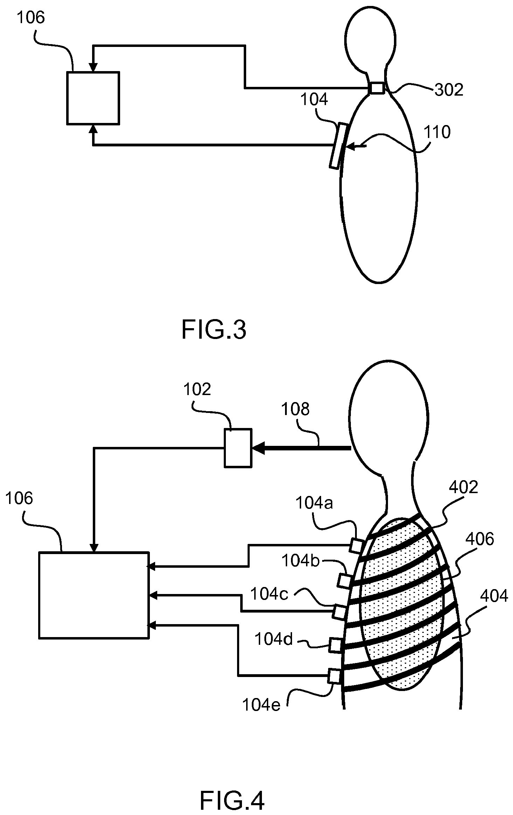

Figure 3 shows a third example of a system used to detect lung abnormalities which uses an actuator 302. In uncooperative or unconscious subjects, it may not be feasible to ask the subject to speak the pre-defined words. In such cases, an acoustic actuator 302 (e.g. a speaker) can be placed over the trachea or alternatively inserted into the mouth of the subject to transmit a first acoustic signal 108, such as a standard sound signal, through the lung airways. The transmitted sounds can then be detected using an arrangement of second sensors 104 placed over the chest wall. The two sounds are then processed and compared to detect any lung pathologies.

However, using an acoustic actuator 302 also provides advantages for subjects which are not uncooperative or unconscious.

The intensity and frequency of the acoustic signal 108 generated through an acoustic actuator 302 can be controlled and may serve as benchmark for analysis of sounds recorded through the arrangement of sensors 104. This removes one source of potential error in analysis.

It is possible to reproduce the same sound at repeated intervals (e.g. during the same examination or from one examination to the next).

It is also possible to produce even more specific sound frequencies which are more suitable for analysis of pathology than those which can be created by the subject's own vocal cords.

Comparing the first acoustic signal 108 and the second acoustic signal 110 based around specific frequency bands may be more suitable to detect specific pathologies (e.g. providing a multiplicity of frequencies at the same time). For example, the first acoustic signal 108 could be a frequency sweep i.e. sequentially generating a single frequency and changing this in time. The advantage of a single frequency is that it is possible to generate resonances at specific frequencies, depending upon the physical dimensions of the lung airways (like air in an organ pipe). By evaluating the resonances in the frequency sweep it may be possible to determine for example where there is a build-up of fluid or mass in the lung (e.g. the absence of higher frequency resonances may indicate that the smaller bronchi are blocked).

Figure 4 shows an example of an arrangement of second sensors 104a-104e. The arrangement of second sensors 104a-104e comprises a microphone array patch. The microphone array patch may be logically clustered and placed over the chest wall such that each microphone covers underlying specific anatomical regions of the lungs 406 (such lobes and bronchopulmonary segments). This clustering would require alignment with the subject's anatomy. Surface anatomy of each specific bronchopulmonary segment or lung lobe would be associated with detected ribs 402 and intercostal spaces 404 as well as key vertical lines e.g. mid-clavicular line and mid-axillary line (information which could be encoded by placement of specific elements of the patch over such landmarks).

Otherwise a regression model using chest circumference, height and biological gender as parameters may indicate cluster positions. For example, a standard map of skin surface extents of bronchopulmonary segments and pulmonary lobes would be constructed for each gender assuming a standard height (say, hs) and chest circumference (say, ccs), and if the subject's measured height and chest circumference are hp and ccp respectively; then the map would be anisotropically scaled by hp/hs in the vertical direction and ccp/ccs in the horizontal direction. The microphone array may comprise multiple segments coupled to each other by some flexible fabric.

If the pieces covering two sides of the chest (or placed above a lung lobe or bronchopulmonary segment) are disjoint, no microphone is guaranteed to be placed over the midline structures like heart or mediastinum. That is to say, no determination of the anatomical structures would be required, as placement of the microphone patches would address the issue. However, if the assembly comes in a single piece, some microphones may be placed over those structures and would need to be switched off/ignored. Similarly, the lobes/bronchopulmonary segments would need to be demarcated. The height and chest circumference may be used to impute the chest wall anatomy and as such determine which microphones are placed above which structures and act accordingly.

The term lung region as used in this document may relate to:
left and right sides;
placement above pulmonary lobes;
placement over bronchopulmonary segments; or
placement over relatively central regions (regions physically closer to the larger divisions of bronchi, on chest surface just lateral to the midline at the 3rd to 5th intercostal spaces) vs. peripheral regions (e.g. at the apex and base of lungs with the smaller bronchi).

After the bronchi enter the lung, they divide repeatedly into smaller branches to ultimately terminate in alveoli (tracheobronchial tree ∼23 divisions ("generations")). The bigger conducting bronchi are near the lung hilum while the terminal and respiratory bronchioles are near the periphery. Though it would be impractical to try to diagnose pathologies of all levels of bronchi and bronchioles, conditions of a small number or classes (each comprising a few levels of bronchi or bronchioles) should be still discriminable. The pulmonary segments may also comprise a number of hierarchical levels which may vary from 2-5 (arbitrary small integer).

The ribs 402 and intercostal spaces 404 between the ribs cause respectively different effects on the intensity of sounds transmitted though the lungs, with the ribs 402 causing attenuation of sounds from within the chest and augmentation of sounds originating from the surface of the chest, while the intercostal spaces 404 cause little or no attenuation or augmentation of such sounds. Since they both produce different changes in the signal, it is desirable to classify the signals by whether they are aligned with the ribs 402 or with the intercostal spaces 404.

Figure 4 shows a microphone array comprising five microphones 104a, 104b, 104c, 104d and 104e. The first, third and fifth microphones, 104a, 104c and 104e, are placed above intercostal spacings 404, whereas the second and fourth microphones, 104b and 104d, are placed above the ribs 402. Only signals from the microphones which are placed on the intercostal spaces 404 are compared to the first acoustic signal 108 from a first sensor 102, as the signal from the intercostal spaces 404 has only been attenuated by being transmitted through the trachea and lungs 406 (and possibly the lung abnormality), and has not been attenuated or augmented by the ribs 402.

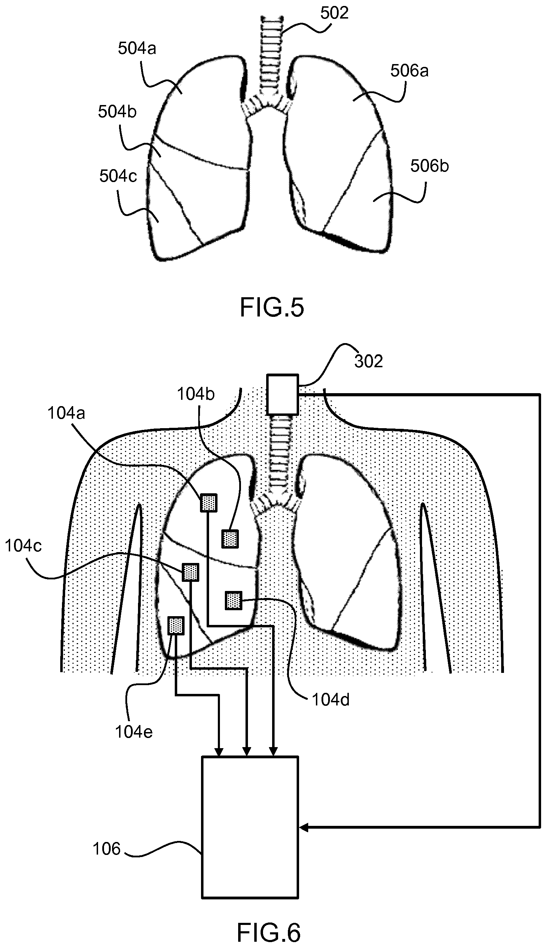

Figure 5 shows a representation of human lungs. Sounds may be transmitted through the trachea 502 into the lungs. Anatomically both the lungs may be further subdivided into constituting lobes. These lobes can be marked on the chest wall using anatomical landmarks described in the literature, such as the superior lobe 504a, the middle lobe 504b and the inferior lobe 504c on the right lung, and the superior lobe 506a and the inferior lobe 506b on the left lung. On the right lung, the superior lobe 504a and the middle lobe 504b are separated by the horizontal fissure and the middle lobe 504b and the inferior lobe 504c are separated by the oblique fissure. On the left lung, the superior lobe 506a and the inferior lobe 506b are separated by the oblique fissure.

The lobes may be further divided in bronchopulmonary segments. It is possible to use surface anatomy markers to map the bronchopulmonary segments to the chest wall as well. As the airways (and the alveoli connected to them) are organized in a tree-like structure, any obstruction of the airway typically involves a bronchopulmonary segment (or the part thereof). As these structures are mappable on the chest surface, locations of the abnormal whispered pectoriloquy can be used to identify possible location or segment of lung pathology as well.

Figure 6 shows a fourth example of a system used to detect lung abnormalities which uses a microphone array 104 and an acoustic actuator 302. The microphone array 104 is placed above one lung initially. There is also an acoustic actuator 302 placed on the throat above the trachea 502 in order to create a first acoustic signal 108. The actuator 302 can produce a plurality of signals which can be chosen beforehand. The first acoustic signal 108 from the actuator is transmitted through the trachea 502, into the lungs and through the intercostal spaces (ICS). Some microphones may be placed on the ribs, such as microphone number two 104b and number four 104d in Figure 6, and therefore the signal from these microphones is not analyzed by the processor 106. The signals from the actuator 302 and the microphones which are placed on the intercostal spaces, 104a, 104c and 104e, are then analyzed by the processor 106 to detect a lung abnormality.

If there is a lung abnormality, the placing of each microphone in the microphone array 104 can be used to determine which lung region each microphone is sensing. The signals from each lung region can then be compared to each other to determine where the lung abnormality is.

The microphone array 104 can be placed on the other lung thereafter to determine whether the other lung may also have an abnormality. Alternatively, if no lung abnormality was found on the first lung, the second lung can then be checked without sensing the segments.

It is also possible to use a microphone array 104 which spans both lungs, or a smaller microphone array which only spans a lobe and is sequentially placed in all the other lobes to determine which, if any, lobe has an abnormality.

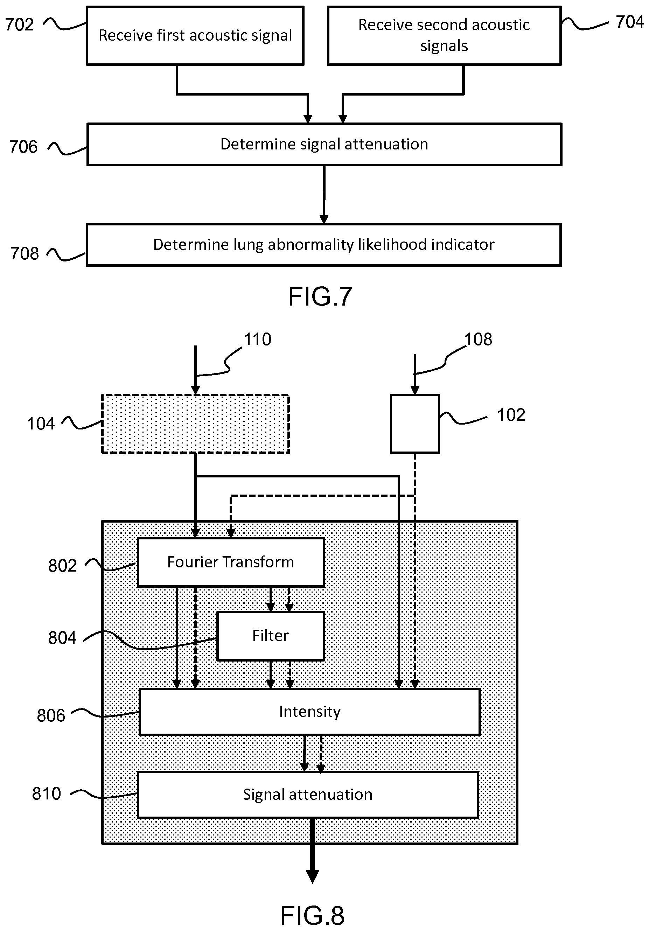

Figure 7 shows a first example of how to determine a lung abnormality likelihood indicator. The lung abnormality likelihood indicator indicates the likelihood of a subject having a lung abnormality. First, a first acoustic signal from a first sensor is received which has been generated by, or applied to, the subject in step 702. Second acoustic signals from an arrangement of one or more second sensors are also received from the lungs of the subject in step 704, wherein the second acoustic signals comprise attenuated versions of the first acoustic signals after passing through the lungs of the subject. The respective signal attenuation between the first acoustic signal and at least a subset of the plurality of second acoustic signals is determined, in step 706. The signal attenuations are thus processed, thereby determining the lung abnormality likelihood indicator in step 708.

The lung abnormality likelihood indicator may be used to help a clinician determine whether a subject has a lung abnormality. The lung abnormality likelihood indicator may be one or more of, but not limited to: a percentage confidence level (e.g. "70%" or "90% likely to have a lung consolidation on the inferior right lobe"), a yes/no indication or a color mapping to different lung regions (e.g. a map of the lungs, wherein a lung region is shaded red when it is likely to have a lung abnormality, yellow when unsure and green when unlikely)

Figure 8 shows an example of how a processor 106 may calculate the signal attenuation. Various pathologies of the lung may be detected through analysis of the sounds vocalized by a subject (e.g. recorded by the standalone microphone) or generated through other means and recorded with an arrangement of sensors 104 over the chest wall. In order to analyze the signals, various parameters of each of the signal may be determined.

One method is to compute the ratio of the intensity of the signal from the arrangement of sensors 104 and the intensity of the signal from the first sensor 102 for same word in each intercostal spacing. If the ratio is beyond a normal expected band or range, e.g., if it is less than a lower threshold or greater than an upper threshold, the possibility of a pathology is indicated. A model trained on a representative database of normal and abnormal pathologies can be used for such classification.

Another method is to compute the frequency spectrum or frequency response (such as by using a Fourier Transform or Fast Fourier Transform) of the two sets of signals and compare frequency features by comparing the spectra of the signals, or by matching previously generated patterns or by using a pre-trained machine learning model.

Alternatively, the two sets of signals may be subjected to different low pass (e.g. ≤ 150 Hz), high pass (e.g. ≥ 2 kHz), and band pass filters (e.g. 200 Hz - 1 kHz). The actual frequencies used will of course be implementation dependent. The outputs can then be cross-correlated, after normalizing intensities. A change in the frequency characteristics is used to indicate the possibility of a pathology. For example, depending on the acoustic impedance of the subject's chest wall and the arrangement of sensors 104, as well as the sensitivities of different sensors, normally a signal below 150 Hz may have approximately a 2 dB reduction in intensity while recorded from the chest wall as compared to a recording from a standalone microphone. A signal above 2 kHz may have approximately an 8 dB reduction in intensity. In the case of a consolidated lung region, each of the two frequency bands may show an intensity reduction of around 5 dB.

Based on the analysis of one or more of the intensity and the frequency of the first acoustic signal 108 and the second acoustic signal 110, a signal attenuation can be found.

Figure 8 shows three different ways in which the signal attenuation may be found. The first method involves finding the intensity difference between the first acoustic signal 108 and the second acoustic signal 110 in step 806 and calculating the signal attenuation, step 810, from the difference in intensity. This could be done by measuring the difference between peak intensities of both signals, or by finding the difference between the average intensities. Another method involves first finding a frequency response of each of the signals, in step 802, and then finding the intensity difference between them, in step 806. The intensity difference may be found by a convolution between the frequency responses of the signals. The third method involves also finding the frequency response in step 802, however in this case a filter is also applied, in step 804, so that only certain frequencies are measured. An intensity difference between the filtered frequency responses of the signals can then be found, in step 806, by either measuring the difference in peak intensities, measuring the difference in average intensities or by a convolution between the filtered frequency responses of the signals. The signal attenuation can then be calculated, step 810, from any of the above methods.

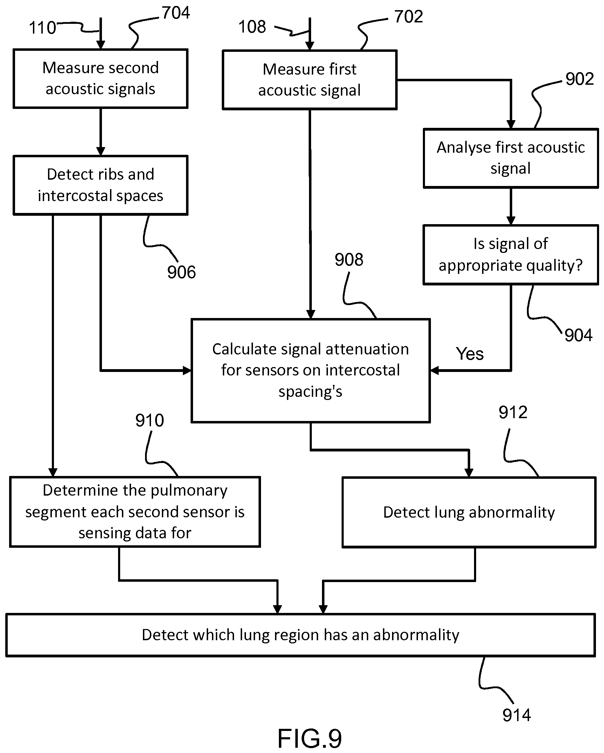

Figure 9 shows an example of how to determine if a pulmonary segment has an abnormality. The method comprises first measuring a first acoustic signal 108, step 702, and a second acoustic signal 110, step 704. The first acoustic signal is first analyzed, step 902, and it is determined whether the signal is of appropriate quality, step 904, in order to avoid analyzing unwanted acoustic signals such as someone else in the room speaking.

It is then determined whether each sensor is on a rib or on an intercostal spacing, step 906. The processor 106 then calculates a signal attenuation, step 908, based on the first acoustic signal (when it has been determined to be of appropriate quality) and the second acoustic signals from the second sensors which are on the intercostal spacing's. From the signal attenuation, it can be determined whether the subject has a lung abnormality, step 912.

The lung region above which each second sensor is located, and therefore to which the sensing data relates, can be determined, in step 910, by, for example, the positioning of the second sensors on the body of the subject. Based on detecting a lung abnormality and determining which second sensors are above which lung regions, a lung region with a lung abnormality may be determined in step 914.

In addition, with the analysis of the signals from spatial clusters (e.g. groups of sensors mapped to lung regions) further analysis may be performed.

For example, the intensity and waveform of signals from the central and peripheral zones (as described above) could be compared. The signal intensity will be reduced in the periphery with change in waveform (which is responsible for loss of 'clarity'). A machine learning model generated by training with sounds from both zones from physiologically normal recordings and from subjects with disease (e.g. lung consolidation) can be used for comparisons and to identify abnormalities. Deviation from a normal pattern beyond an acceptable limit could be determined using a pre-trained model.

With sensors identified to be above specific pulmonary lobes or bronchopulmonary segments, if any abnormality is found, the spatial clusters could be analyzed individually to determine if the pathology is confined to any pulmonary lobe or bronchopulmonary segment. Optionally, once a positive localization is detected, signals from clusters neighboring the affected clusters would be analyzed with different thresholds.

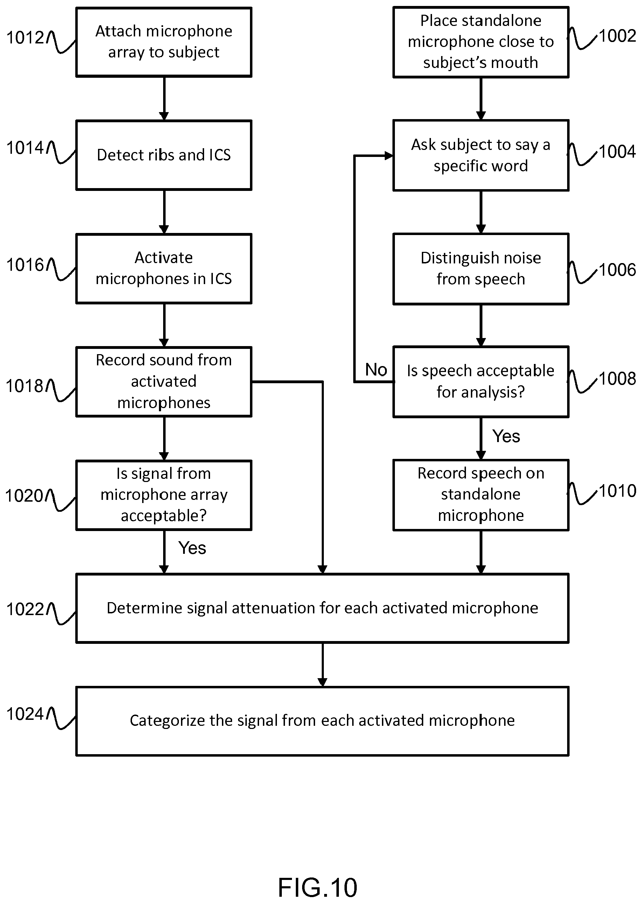

Figure 10 shows an example of how to categorize the attenuated acoustic signals. A microphone array patch (e.g. a modified capacitive micromachined ultrasound transducer, CMUT, array) is attached to a subject's chest wall or back, in step 1012 and a separate standalone microphone is placed close to the subject's mouth in step 1002.

The subject is then asked to say a few specific words (e.g. ninety-nine), in step 1004. There may be a set of specific words sufficiently covering low and high frequency sounds, and from languages appropriate for proper communication to the subject. The words chosen are thus based on low-frequency and high-frequency components. In addition, the subject may be asked to whisper some words and speak some words as he or she would in the normal course of conversation.

The signal from the standalone microphone is used to distinguish between silence or background noise and speech, based on intensity and frequency properties of the recorded signal, in step 1006. The intensity and frequency properties of normal speech are well known in the literature.

The speech signal is further analyzed and identified words matched with existing patterns (using a pre-trained model) to determine whether the words spoken by the subject were correct and the intensity profile was acceptable for further analysis, in step 1008. If not acceptable, the subject could be asked to repeat the word or say a different word. The model may be generated by training a machine learning framework with annotated recordings of spoken words (read from a written sample or otherwise communicated to the subject) from a population of subjects, both with and without pulmonary pathologies.

The microphone array patch is used to detect the ribs and intercostal spaces, in step 1014. Based on identified areas, the microphones in the intercostal spaces are activated, in step 1016, to listen to sounds getting transmitted through the chest wall.

The sound recording from the chest wall is performed through the microphone array patch, in step 1018. The sound from the patch during speech is analyzed (intensity and frequency profile compared with recording from the standalone microphone, without inferring normalcy or diseased state) to determine whether the received signal is appropriate and acceptable for further analysis, in step 1020. This analysis will help false inference that may result from loose or other improper attachment of the patch generating spurious sounds or a poor quality signal.

The sound recorded from the patch during speech is then correlated with the sound recorded from the standalone microphone to determine the signal attenuation, in step 1022. The basic comparison may involve any of three different aspects; intensity, frequency and spatial distribution. The signal attenuation can then be calculated from this comparison.

The sounds recorded in the different intercostal spaces by the patch microphones may also be compared with each other. Based on the signal attenuation, the recorded sounds can be categorized into four broad categories, in step 1024: normal resonant sounds; increased vocal resonant; diminished vocal resonant; and absent vocal resonant. This classification can be done using a single method or by combining multiple methods.

In this way, the processor determines a vocal resonance level and this may be used to identify a type or range of possible types of lung abnormality.

Classification of the recorded sounds based on a pre-defined or dynamic thresholding of sound intensity is one way to implement this categorization. Another implementation could involve machine learning algorithms. A category of the respiratory sound recorded in a particular intercostal space can be indicated to the user using audio-visual indicators. The categorization of the second acoustic signals can be used by a processor to determine the presence and location of a lung abnormality, or to aid a clinician in determining the presence and location of a lung abnormality.

In the cases of lung pathologies, it is important to compare the respiratory sounds from to the two sides of the chest to determine changes in sound properties. The respiratory sounds recorded from one side of the chest wall will be then compared with the sounds recorded from the other side, in order to determine differences in intensity and quality of the recorded sounds. This comparison can be then used to find out the side with abnormal respiratory sounds. An algorithm trained on a representative database of normal and abnormal sounds can then be used to classify whether sounds recorded from an intercostal space are abnormal.

The skilled person would be readily capable of developing a processor for carrying out any herein described method. Thus, each step of a flow chart may represent a different action performed by a processor, and may be performed by a respective module of the processing processor.

As discussed above, the system makes use of processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A system for detecting lung abnormalities in a subject comprising:
a first sensor (102) for sensing a first acoustic signal (108) generated by, or applied to, the subject;
an arrangement of one or more second sensors (104) for detecting a plurality of second acoustic signals (110) from the lungs of the subject, wherein the second acoustic signals comprise attenuated versions of the first acoustic signal after passing through the lungs of the subject; and
a processor (106) for detecting lung abnormalities, wherein the processor is configured to:
determine a respective signal attenuation between the first acoustic signal (108) and at least a subset of the plurality of second acoustic signals (110); and
process the signal attenuations thereby to detect a lung abnormality.

2. A system as claimed in claim 1, comprising an acoustic actuator (302) for generating the first acoustic signal (108), wherein the acoustic actuator is for application in the vicinity of the larynx of the subject, and wherein the first sensor (102) is adapted to sense the first acoustic signal directly or to sense the first acoustic signal based on a drive signal provided to the acoustic actuator.

3. A system as claimed in claim 1, wherein the first acoustic signal comprises speech generated by the subject, wherein the first sensor (102) is one of: a standalone microphone (202), a throat microphone, an accelerometer, a wave radar, a Doppler radar or an ultrasound sensor.

4. A system as claimed in claim 3, wherein the processor is configured to detect a presence or absence of speech in the first acoustic signal based on frequency and intensity analysis of the first acoustic signal, and is further configured to identify predetermined words or word sequences.

5. A system as claimed in claim 4, wherein the processor (106) comprises a trained machine learning speech recognition network for identifying said predetermined words or word sequences.

6. A system as claimed in claim 4 or 5, wherein said arrangement of one or more second sensors comprises a microphone patch array comprising a plurality of said second sensors.

7. A system as claimed in claim 6, wherein the processor (106) is further configured to:
determine placement of the one or more second sensors based on the plurality of second acoustic signals, wherein the placement comprises rib-alignment or intercostal space-alignment;
determine a subset of the one or more second sensors that are intercostal space-aligned; and
determine a subset of the plurality of second acoustic signals generated by the subset of the one or more second sensors that are intercostal space-aligned.

8. A system as claimed in claim 7, wherein the processor is configured to:
determine the signal attenuation, at least for said subset of the plurality of second acoustic signals, as a ratio between the intensity of the respective second acoustic signal and the intensity of the first acoustic signal, for a same word or word sequence.

9. A system as claimed in claim 7, wherein the processor is configured to:
determine a vocal resonance level, at least for said subset of the plurality of second acoustic signals, thereby to identify a type or range of possible types of lung abnormality.

10. A system as claimed in any one of claims 6 to 9, wherein the processor is configured to:
determine a frequency spectrum of the first acoustic signal and at least said subset of the plurality of second acoustic signals;
normalize the amplitudes of the frequency spectra of the first and second acoustic signals; and
perform cross correlations between components of the normalized frequency spectrum of the first acoustic signal and the corresponding components of the normalized frequency spectra of the second acoustic signals.

11. A system as claimed in any one of claims 6 to 10, wherein the processor (106) is further configured to:
for a plurality of lung regions, determine clusters of the second sensors which are associated with each lung region, based on the placement of each of the second sensors, wherein a second sensor is associated with the lung regions to which it is closest; and
for a detected lung abnormality, determine the position of the lung abnormality based on the clusters of second sensors at which the lung abnormality is detected.

12. A system as claimed in claim 11, wherein the processor is further configured to:
classify at least each of said second sensors corresponding to said subset of the plurality of second acoustic signals as associated with a central part of a lung region or a peripheral part of a lung region; and
compare the second acoustic signals between a sensor classified as associated with the central part of a lung region and a sensor classified as associated with the peripheral part of that lung region.

13. A system as claimed in claim 12, wherein the processor comprises a machine learning model trained using a database of sounds in respect of normal and abnormal pathologies, and in respect of sensor signals for central parts of the lung regions and for peripheral parts of the lung regions.

14. A system as claimed in any one of claims 6 to 13, wherein the processor is configured to compare second acoustic signals for opposite sides of the chest at least for said subset of the plurality of second acoustic signals.

15. A method for determining a lung abnormality likelihood indicator, wherein the lung abnormality likelihood indicator indicates the likelihood of a subject having a lung abnormality, the method comprising:
receiving signals from a first sensor (102) for sensing a first acoustic signal (108) generated by, or applied to, the subject;
receiving signals from an arrangement of one or more second sensors (104) for detecting a plurality of second acoustic signals (110) from the lungs of the subject, wherein the second acoustic signals comprise attenuated versions of the first acoustic signals after passing through the lungs of the subject; and
determining the lung abnormality likelihood indicator by:
determining respective signal attenuations between the first acoustic signal (108) and at least a subset of the plurality of second acoustic signals (110); and
processing the signal attenuations thereby to determine the lung abnormality likelihood indicator.

16. A computer program comprising code means for implementing the method of claim 15 when said computer program is run on a processing system.
